# EUROPEAN PATENT APPLICATION

(11) **EP 4 674 355 A1**
(43) Date of publication of application: **07.01.2026**
(21) Application number: 24763567.5
(22) Date of filing: 08.02.2024
(51) Int. Cl.: A61B 8/08

(54) **ULTRASONIC DIAGNOSTIC DEVICE AND METHOD FOR CONTROLLING ULTRASONIC DIAGNOSTIC DEVICE**

(30) Priority: 02.03.2023 JP 2023031574
(71) Applicant: FUJIFILM Corporation, Tokyo 106-8620 (JP)
(72) Inventor: TAKEMOTO Kosei, Tokyo 106-8620 (JP)
(74) Representative: Klunker IP Patentanwälte PartG mbB
(86) International application number: PCT/JP2024/004334
(87) International publication number: WO 2024/181083

(57) **Abstract**

An ultrasonic diagnostic apparatus includes: a lesion detection unit (26) that detects a suspected lesion region in a mammary gland region of a subject based on an ultrasonic image in which the mammary gland region is imaged; a mask data creation unit (27) that creates mask data of the detected suspected lesion region; an exclusion region setting unit (28) that sets an exclusion region to be excluded from a target of a glandular tissue component evaluation based on the mask data; and an evaluation unit (29) that performs the glandular tissue component evaluation on an evaluation target region obtained by excluding the exclusion region from the mammary gland region.

## Description

### BACKGROUND OF THE INVENTION

### 1. Field of the Invention

The present invention relates to an ultrasonic diagnostic apparatus used for an examination of a breast of a subject and a method of controlling an ultrasonic diagnostic apparatus.

### 2. Description of the Related Art

In related art, in the medical field, an ultrasonic diagnostic apparatus using ultrasonic images is put into practical use. In general, the ultrasonic diagnostic apparatus comprises an ultrasonic probe provided with a transducer array and an apparatus body connected to the ultrasonic probe, in which an ultrasonic beam is transmitted from the ultrasonic probe toward a subject, an ultrasonic echo from the subject is received by the ultrasonic probe, and a reception signal is electrically processed to generate the ultrasonic image.

A composition of a fat tissue and a mammary gland tissue in a breast varies depending on a person, but an anatomical structure of the breast is common, and a primary lactiferous duct branches into extralobular ducts, which in turn connect to numerous lobules, in the mammary gland tissue. Stroma is present around the lobules, and mammary gland tissue is composed of the lobules together with the stroma.

It is known that two types of stroma exist around the lobules, that is, perilobular stroma and edematous stroma. The perilobular stroma exists along a structure from the lobule to the mammary duct, and includes many collagen fibers. On the other hand, the edematous stroma fills the spaces between the perilobular stroma, is rich in extracellular matrix, with a mixture of collagen fibers and fat, and contains fewer collagen fibers as compared to the perilobular stroma.

In recent years, the concept of individualized risk management for patients has become widespread, but it is known that a ratio of the mammary gland region within the breast, especially a high-density mammary gland, is a risk factor for cancer. The ratio of the mammary gland region in the breast can be measured by using a mammography apparatus.

Further, in Su Hyun Lee et al. "Glandular Tissue Component and Breast Cancer Risk in Mammographically Dense Breasts at Screening Breast US", Radiology, Volume 301, October 1, 2021, it is reported that a cancer is likely to occur in a case in which a ratio of a glandular tissue component (GTC) region including mammary ducts, lobules, and perilobular stroma in the mammary gland region is high even though the mammary gland region is almost the same. Stated another way, a ratio of the GTC region in the mammary gland region may be a risk factor, in addition to the ratio of the mammary gland region in the breast. This means that a patient with less advanced atrophy of the lobule has a higher risk.

However, in the mammography apparatus, the perilobular stroma and the edematous stroma cannot be distinguished from each other, and the entire mammary gland tissue is observed as whitish, and as a result, the ratio of the GTC region in the mammary gland region cannot be measured.

JP2021-185970A discloses an apparatus that extracts a suspected lesion region in a mammary gland region, which is a region suspected to have a lesion, from an ultrasonic image.

### SUMMARY OF THE INVENTION

However, the ultrasonic diagnostic apparatus of JP2021-185970A is intended to detect the suspected lesion region in the mammary gland region, and is not interested in evaluating the GTC region. Therefore, there is an issue in that the risk of cancer in the mammary gland region cannot be considered in detail.

In addition, since both the GTC region and the suspected lesion region are depicted as low-echo regions, that is, low-brightness regions in the ultrasonic image, in a case in which the GTC region is manually evaluated as disclosed in Su Hyun Lee et al. "Glandular Tissue Component and Breast Cancer Risk in Mammographically Dense Breasts at Screening Breast US", Radiology, Volume 301, October 1, 2021, the user, such as a doctor, needs to determine the GTC region and the suspected lesion region, and thus it is difficult to evaluate the GTC region with high accuracy, and there is a case in which the user cannot consider the risk of cancer in the mammary gland region with high accuracy.

The present invention has been made in order to solve such an issue in the related art, and an object of the present invention is to provide an ultrasonic diagnostic apparatus that enables a user to consider a risk of cancer in a mammary gland region of a subject with high accuracy even in a case in which a suspected lesion region is present.

It is possible to achieve the above-described object with the following configurations.
[1] An ultrasonic diagnostic apparatus comprising: a lesion detection unit that detects a suspected lesion region in a mammary gland region of a subject based on an ultrasonic image in which the mammary gland region is imaged; a mask data creation unit that creates mask data of the suspected lesion region detected by the lesion detection unit; an exclusion region setting unit that sets an exclusion region to be excluded from a target of a glandular tissue component evaluation based on the mask data; and an evaluation unit that performs the glandular tissue component evaluation on an evaluation target region obtained by excluding the exclusion region set by the exclusion region setting unit from the mammary gland region.
[2] The ultrasonic diagnostic apparatus according to [1], in which the evaluation unit classifies the evaluation target region into a low-echo region and a high-echo region based on a predetermined brightness threshold value, and outputs a ratio between the number of pixels occupied by the low-echo region and the number of pixels occupied by the high-echo region as a result of the glandular tissue component evaluation.
[3] The ultrasonic diagnostic apparatus according to [1], in which the evaluation unit determines a category of a glandular tissue component in the mammary gland region based on the ultrasonic image including the evaluation target region, and outputs the category as a result of the glandular tissue component evaluation.
[4] The ultrasonic diagnostic apparatus according to [3], in which the evaluation unit determines the category of the glandular tissue component using a trained model that has been trained through machine learning based on a plurality of training data each of which includes the ultrasonic image in which the mammary gland region is imaged and the category of the glandular tissue component in the mammary gland region.
[5] The ultrasonic diagnostic apparatus according to any one of [1] to [4], further comprising: a monitor; and a display control unit that displays the ultrasonic image on the monitor, in which the display control unit highlights the exclusion region set by the exclusion region setting unit on the monitor.
[6] The ultrasonic diagnostic apparatus according to [5], in which the display control unit highlights the exclusion region with respect to the evaluation target region on the monitor.
[7] The ultrasonic diagnostic apparatus according to [5], in which the display control unit displays the exclusion region on the monitor in a color or a form in accordance with a reliability degree of the detection of the suspected lesion region performed by the lesion detection unit.
[8] The ultrasonic diagnostic apparatus according to [5], in which the display control unit displays a dialog for confirming with a user whether to correct or delete the exclusion region on the monitor.
[9] The ultrasonic diagnostic apparatus according to any one of [1] to [8], in which the exclusion region setting unit does not set the exclusion region in a case in which the mask data is smaller than a predetermined first size threshold value.
[10] The ultrasonic diagnostic apparatus according to any one of [1] to [9], in which the evaluation unit does not perform the glandular tissue component evaluation in a case in which the mask data is larger than a predetermined second size threshold value.
[11] The ultrasonic diagnostic apparatus according to any one of [5] to [7], in which the evaluation unit performs the glandular tissue component evaluation on the mammary gland region that does not exclude the exclusion region, in addition to the glandular tissue component evaluation on the evaluation target region, and the display control unit displays a result of the glandular tissue component evaluation on the evaluation target region and a result of the glandular tissue component evaluation on the mammary gland region that does not exclude the exclusion region on the monitor.
[12] The ultrasonic diagnostic apparatus according to any one of [1] to [11], in which the lesion detection unit detects the suspected lesion region using a trained model that has been trained through machine learning based on a plurality of training data each of which includes the ultrasonic image in which the mammary gland region including the suspected lesion region is imaged.
[13] The ultrasonic diagnostic apparatus according to any one of [1] to [11], in which the lesion detection unit detects the suspected lesion region by image-analyzing the ultrasonic image.
[14] The ultrasonic diagnostic apparatus according to any one of [1] to [13], in which the ultrasonic image is a three-dimensional ultrasonic image, and the evaluation unit performs the glandular tissue component evaluation based on the three-dimensional ultrasonic image.
[15] A method of controlling an ultrasonic diagnostic apparatus, the method comprising: detecting a suspected lesion region in a mammary gland region of a subject based on an ultrasonic image in which the mammary gland region is imaged; creating mask data of the detected suspected lesion region; setting an exclusion region to be excluded from a target of a glandular tissue component evaluation based on the mask data; and performing the glandular tissue component evaluation on an evaluation target region obtained by excluding the exclusion region from the mammary gland region.

According to the aspects of the present invention, the ultrasonic diagnostic apparatus comprises: the lesion detection unit that detects the suspected lesion region in the mammary gland region of the subject based on the ultrasonic image in which the mammary gland region is imaged; the mask data creation unit that creates the mask data of the suspected lesion region detected by the lesion detection unit; the exclusion region setting unit that sets the exclusion region to be excluded from the target of the glandular tissue component evaluation based on the mask data; and the evaluation unit that performs the glandular tissue component evaluation on the evaluation target region obtained by excluding the exclusion region set by the exclusion region setting unit from the mammary gland region, so that the user can consider a risk of cancer in the mammary gland region of the subject with high accuracy even in a case in which a suspected lesion region is present.

### BRIEF DESCRIPTION OF THE DRAWINGS

Fig. 1 is a block diagram showing a configuration of an ultrasonic diagnostic apparatus according to an embodiment of the present invention.
Fig. 2 is a block diagram showing an internal configuration of a transmission-and-reception circuit according to the embodiment.
Fig. 3 is a block diagram showing an internal configuration of an image generation unit according to the embodiment.
Fig. 4 is a diagram showing an ultrasonic image obtained by imaging a mammary gland region of a subject.
Fig. 5 is a diagram showing an example of mask data.
Fig. 6 is a diagram showing the ultrasonic image in which an exclusion region is set.
Fig. 7 is a diagram showing a display example of an evaluation result of a GTC evaluation.
Fig. 8 is a flowchart showing an operation according to the embodiment.
Fig. 9 is a diagram showing another example of the mask data.
Fig. 10 is a diagram showing another display example of the evaluation result of the GTC evaluation.

### DESCRIPTION OF THE PREFERRED EMBODIMENTS

Hereinafter, embodiments of the invention will be described with reference to the accompanying drawings.

The following configuration requirements are described based on a representative embodiment of the present invention, but the present invention is not limited to such an embodiment.

In the present specification, a numerical range represented by "to" means a range including numerical values described before and after "to", both ends inclusive, as a lower limit value and an upper limit value.

In the present specification, "same" and "identical" include an error range that is generally allowed in the technical field.

### [Embodiment]

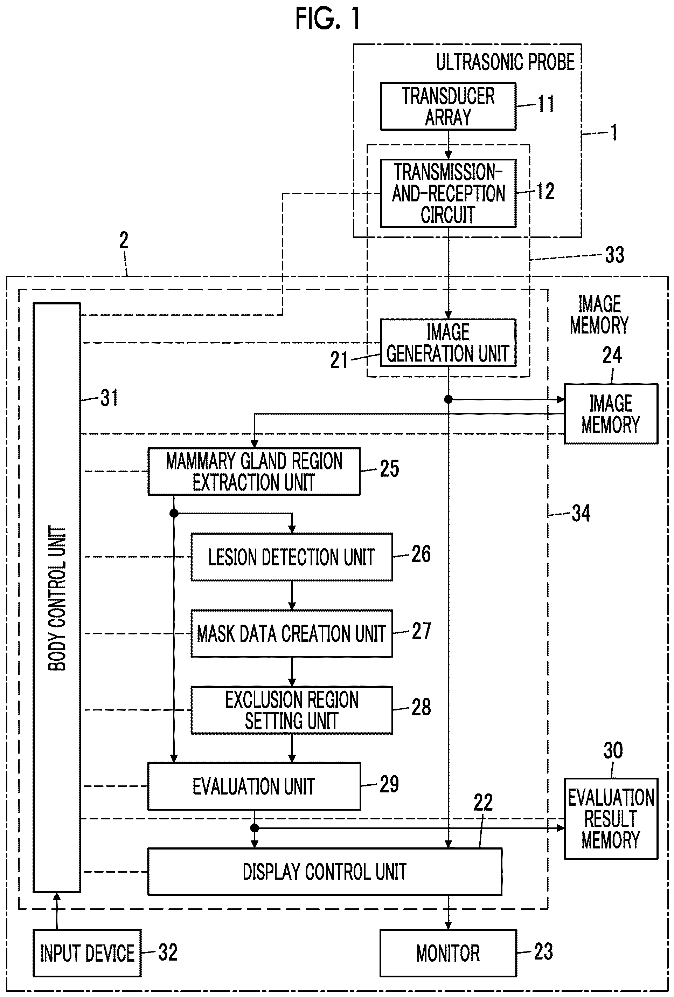

Fig. 1 shows a configuration of an ultrasonic diagnostic apparatus according to the embodiment of the present invention. The ultrasonic diagnostic apparatus comprises an ultrasonic probe 1 and an apparatus body 2. The ultrasonic probe 1 and the apparatus body 2 are wired-connected to each other via a cable (not shown).

The ultrasonic probe 1 includes a transducer array 11 and a transmission-and-reception circuit 12 connected to the transducer array 11.

The apparatus body 2 includes an image generation unit 21 connected to the transmission-and-reception circuit 12 of the ultrasonic probe 1, a display control unit 22 and a monitor 23 are connected sequentially to the image generation unit 21, and an image memory 24 is connected to the image generation unit 21. Further, a mammary gland region extraction unit 25 is connected to the image memory 24. A lesion detection unit 26, a mask data creation unit 27, and an exclusion region setting unit 28 are connected sequentially to the mammary gland region extraction unit 25. In addition, an evaluation unit 29 is connected to the mammary gland region extraction unit 25 and the exclusion region setting unit 28. The display control unit 22 and an evaluation result memory 30 are connected to the evaluation unit 29.

In addition, a body control unit 31 is connected to the image generation unit 21, the display control unit 22, the image memory 24, the mammary gland region extraction unit 25, the mask data creation unit 27, the exclusion region setting unit 28, the evaluation unit 29, and the evaluation result memory 30. An input device 32 is connected to the body control unit 31. The transmission-and-reception circuit 12 and the image generation unit 21 constitute an image acquisition unit 33. The image generation unit 21, the display control unit 22, the mammary gland region extraction unit 25, the lesion detection unit 26, the mask data creation unit 27, the exclusion region setting unit 28, the evaluation unit 29, and the body control unit 31 constitute a processor 34 for the apparatus body 2.

The transducer array 11 of the ultrasonic probe 1 includes a plurality of ultrasonic transducers arranged in a one-dimensional or two-dimensional manner. Each of these transducers transmits an ultrasonic wave in response to a drive signal supplied from the transmission-and-reception circuit 12, receives a reflected wave from a subject, and outputs an analog reception signal. Each transducer is formed by, for example, forming electrodes on both ends of a piezoelectric body consisting of a piezoelectric single crystal represented by lead zirconate titanate (PZT), a polymeric piezoelectric element represented by poly vinylidene di fluoride (PVDF), or a piezoelectric single crystal represented by a lead magnesium niobate-lead titanate (PMN-PT) solid solution.

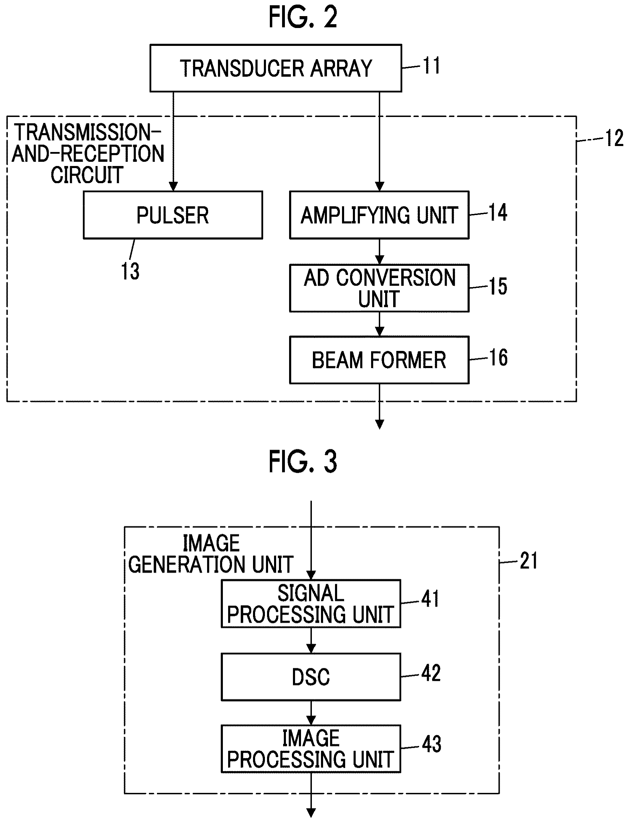

The transmission-and-reception circuit 12 transmits the ultrasonic wave from the transducer array 11 and generates a sound ray signal based on the reception signal acquired by the transducer array 11, under the control of the body control unit 31. The transmission-and-reception circuit 12 includes, as shown in Fig. 2, a pulser 13 connected to the transducer array 11, and an amplifying unit 14, an analog-to-digital (AD) conversion unit 15, and a beam former 16 which are sequentially connected in series to the transducer array 11.

The pulser 13 includes, for example, a plurality of pulse generators, adjusts a delay amount of each drive signal based on a transmission delay pattern selected in accordance with a control signal from the body control unit 31 such that ultrasonic waves to be transmitted from the plurality of transducers of the transducer array 11 form a ultrasonic beam, and supplies the drive signal of which the delay amount has been adjusted, to the plurality of transducers. In this way, in a case in which a pulsed or continuous wave voltage is applied to the electrodes of the transducers of the transducer array 11, the piezoelectric body expands and contracts to generate a pulsed or continuous wave ultrasonic wave from each transducer, and the ultrasonic beam is formed from the combined wave of these ultrasonic waves.

The transmitted ultrasonic beam is reflected by a target, for example, a part of the subject, and an ultrasonic echo propagates toward the transducer array 11 of the ultrasonic probe 1. The ultrasonic echo propagating toward the transducer array 11 in this manner is received by each of the transducers constituting the transducer array 11. In this case, each transducer constituting the transducer array 11 expands and contracts by receiving the propagating ultrasonic echo to generate the reception signal that is an electric signal, and outputs the reception signal to the amplifying unit 14.

The amplifying unit 14 amplifies the signal input from each of the transducers constituting the transducer array 11 and transmits the amplified signal to the AD conversion unit 15. The AD conversion unit 15 converts the signal transmitted from the amplifying unit 14 into digital reception data, and transmits the reception data to the beam former 16. The beam former 16 performs so-called reception focus processing by giving and adding delay with respect to each reception data converted by the AD conversion unit 15, in accordance with a sound velocity or a sound velocity distribution set based on a reception delay pattern selected according to a control signal from the body control unit 31. Through the reception focus processing, a sound ray signal is acquired in which each piece of the reception data converted by the AD conversion unit 15 is phased and added and the focus of the ultrasonic echo is narrowed.

The image generation unit 21 of the apparatus body 2 has, as shown in Fig. 3, a configuration in which a signal processing unit 41, a digital scan converter (DSC) 42, and an image processing unit 43 are sequentially connected in series.

The signal processing unit 41 performs, on the sound ray signal transmitted from the transmission-and-reception circuit 12 of the ultrasonic probe 1, correction of attenuation caused by a distance in accordance with a depth of a reflection position of the ultrasonic wave and then performs envelope detection processing, and thereby generates an ultrasonic image signal (B-mode image signal), which is tomographic image information related to tissues in the subject.

The DSC 42 converts (raster-converts) the ultrasonic image signal generated by the signal processing unit 41 into an image signal in accordance with a normal television signal scanning method.

The image processing unit 43 performs various types of necessary image processing, such as gradation processing, on the ultrasonic image signal input from the DSC 42, and then outputs the signal representing the ultrasonic image to the display control unit 22 and the image memory 24. The signal representing the ultrasonic image generated by the image generation unit 21 in this way will be simply referred to as the ultrasonic image. In addition, the image generation unit 21 can also output the ultrasonic image signal before being processed by the DSC 42 or the ultrasonic image signal immediately after being processed by the DSC 42 to the image memory 24. In this case, the image generation unit 21 can generate the ultrasonic image by reading out these signals from the image memory 24 and performing processing using the DSC 42 or the image processing unit 43.

The image memory 24 is a memory that stores the ultrasonic image generated by the image generation unit 21 under the control of the body control unit 31. For example, the image memory 24 can store a plurality of frames of ultrasonic images generated by the image generation unit 21 in correspondence with diagnosis on a mammary gland region of a breast of the subject.

As the image memory 24, for example, a recording medium such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), or a universal serial bus memory (USB memory), can be used.

The mammary gland region extraction unit 25 detects a breast region of the subject from the ultrasonic image read out from the image memory 24, and extracts the mammary gland region from the detected breast region.

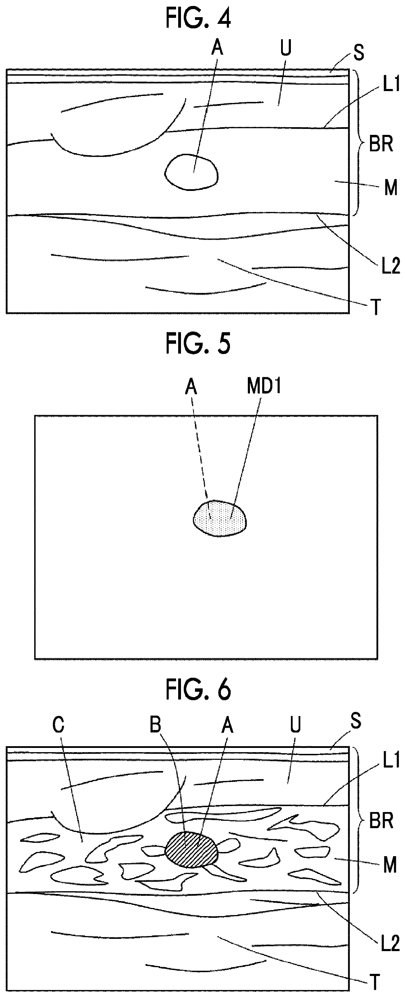

Fig. 4 shows an example of an ultrasonic image U in which the breast of the subject is imaged. The ultrasonic image U is a tomographic image captured by bringing a distal end of the ultrasonic probe 1 into contact with the breast of the subject, in which a skin S of the subject is shown in an upper end of the ultrasonic image U representing a shallowest portion, and a pectoralis major T is shown in a lower portion of the ultrasonic image U representing a deeper portion. The mammary gland region extraction unit 25 can recognize a skin S and a pectoralis major T from the ultrasonic image U and detect a deep region between the skin S and the pectoralis major T as a breast region BR.

As shown in Fig. 4, the mammary gland region extraction unit 25 can recognize a front boundary line L1 located on a shallower side and a rear boundary line L2 located on a deeper side in the detected breast region BR, and can extract a deep region between the front boundary line L1 and the rear boundary line L2 as a mammary gland region M.

In order to detect the breast region BR and to extract the mammary gland region M described above, the mammary gland region extraction unit 25 can perform image recognition using at least one of template matching, an image analysis technique using a feature value, such as adaptive boosting (AdaBoost), support vector machine (SVM), or scale-invariant feature transform (SIFT), or a determination model that has been trained by using a machine learning technique such as deep learning.

The determination model is a trained model that has learned the breast region BR and the mammary gland region M (segmentation) of the breast region BR in a training ultrasonic image obtained by imaging the breast.

The lesion detection unit 26 detects a suspected lesion region A based on the ultrasonic image U in which the mammary gland region M of the subject is imaged. Here, the suspected lesion region A means a region in which a lesion including a so-called tumor is suspected in the mammary gland region M. The lesion detection unit 26 can detect the suspected lesion region A by using, for example, at least one of template matching, an image analysis technique using a feature value, such as AdaBoost, SVM, or SIFT, or a determination model that has been trained by using a machine learning technique such as deep learning. The determination model used here is a trained model that has learned a plurality of lesion parts in the ultrasonic image U in which the breast region BR is imaged.

The mask data creation unit 27 creates mask data MD1 of the suspected lesion region A detected by the lesion detection unit 26 as shown in Fig. 5. Here, the mask data MD1 is data representing a figure surrounding the suspected lesion region A, and can be represented by, for example, matrix data including coordinates of a plurality of vertices of a so-called polygon along a contour of the suspected lesion region A in the ultrasonic image U. In the example of Fig. 5, the mask data MD1 is data having the same size and shape as those of the suspected lesion region A and having the same positional information as the positional information of the suspected lesion region A in the ultrasonic image U.

As shown in Fig. 6, the exclusion region setting unit 28 sets an exclusion region B to be excluded from a target of a glandular tissue component (GTC) evaluation performed by the evaluation unit 29, which will be described later, based on the mask data MD 1 created by the mask data creation unit 27. The exclusion region B is a region that is covered with the mask data MD1 in a case in which the mask data MD1 is superimposed on the ultrasonic image U. In the example of Fig. 6, the exclusion region B is set based on the mask data MD1 having the same size and shape as those of the suspected lesion region A in the ultrasonic image and having the same positional information as the positional information of the suspected lesion region A, so that a region corresponding to the suspected lesion region A in the ultrasonic image U is set as the exclusion region B.

The evaluation unit 29 performs the GTC evaluation on an evaluation target region C obtained by excluding the exclusion region B set by the exclusion region setting unit 28 from the mammary gland region M extracted by the mammary gland region extraction unit 25.

In a case in which the GTC evaluation is performed, the evaluation unit 29 first extracts the GTC region from the evaluation target region C. The GTC region consists of mammary ducts, lobules, and perilobular stroma in the mammary gland region M, and edematous stroma fills a space between the perilobular stroma. Since the edematous stroma is rich in extracellular matrix and contains coexisting fat, in a case of observing the mammary gland region M using the ultrasonic image U, the edematous stroma has a high echo level (high-echo) and appears bright. On the other hand, the mammary duct, the lobule, and the perilobular stroma constituting the GTC region have relatively low echo levels (low-echo), and the brightness is lower than that of the edematous stroma.

Therefore, the evaluation unit 29 can classify the evaluation target region C of the ultrasonic image U into a low-echo region and a high-echo region by, for example, binarizing the evaluation target region C using a brightness threshold value Thb, and can extract the GTC region by distinguishing the GTC region and the edematous stroma from each other in the evaluation target region C.

In addition, a predetermined constant value can be used as the brightness threshold value Thb.

In addition, the evaluation unit 29 may perform edge detection on the GTC region in the ultrasonic image U by image analysis, and may automatically calculate the brightness threshold value Thb based on a change in brightness value in the detected edge portion, that is, a change in brightness value of a plurality of pixels from the inside to the outside of the GTC region. In this way, it is possible to automatically set the brightness threshold value Thb suitable for the ultrasonic image as the image analysis target, and to acquire the binarized image suitable for the ultrasonic image U.

Further, the ultrasonic diagnostic apparatus can be configured such that a histogram of the brightness of the evaluation target region C detected from the ultrasonic image U is created, and the user sets the brightness threshold value Thb by inputting the brightness threshold value Thb from the input device 32 based on the histogram, a binarized image created using the initial value of the brightness threshold value Thb, and the ultrasonic image U generated by the image generation unit 21.

In addition, the evaluation unit 29 can also extract the GTC region using a determination model that has been trained by using a machine learning technique such as deep learning. In this case, for example, a trained model that has learned the GTC region (segmentation) in the evaluation target region C in the training ultrasonic image in which the breast is imaged is used as the determination model.

The evaluation unit 29 calculates a ratio of the GTC region in the evaluation target region C and performs the GTC evaluation based on the calculated ratio of the GTC region. The evaluation unit 29 can calculate a GTC region ratio, for example, by a ratio of the sum of the number of pixels occupied by all the low-echo regions in the evaluation target region C to the number of pixels occupied by the high-echo region in the evaluation target region C in the ultrasonic image U. The evaluation unit 29 can use, for example, the ratio of the GTC region calculated as described above as the evaluation result of the GTC evaluation.

For example, the evaluation unit 29 can determine a category of the GTC region based on the ratio of the GTC region in the evaluation target region C and use the category as the evaluation result of the GTC evaluation.

It is generally known that the lobule atrophies with age, but there are research results that a risk of breast cancer is high in patients in whom the lobule does not atrophy, as disclosed in, for example, "Su Hyun Lee et al. "Glandular Tissue Component and Breast Cancer Risk in Mammographically Dense Breasts at Screening Breast US", Radiology, Volume 301, October 1, 2021". The category of the GTC region represents a degree of progression of the atrophy of the lobule, and can be used as a material for determining the risk of breast cancer.

The evaluation unit 29 can also determine the category of the GTC region using, for example, a trained model that has been trained through machine learning based on a plurality of training data each of which includes the ultrasonic image U in which the mammary gland region M is imaged and the category of the GTC region in the ultrasonic image U, as shown in Fig. 4 or Fig. 6. The association between the ultrasonic image U and the category of the GTC region in the training data can be performed by an expert, such as a skilled doctor.

The evaluation unit 29 can output, as the evaluation result, any one of a plurality of predetermined categories, for example, any one of two categories of Low and High as the category of the GTC region. Low indicates that the lobule atrophy has not progressed as much as in High. Further, the evaluation unit 29 can also output, for example, any one of four categories of Minimal, Mild, Moderate, and Marked as the category of the GTC region. Mild indicates that the atrophy of the lobule is not more advanced than that in Minimal, Moderate indicates that the atrophy of the lobule is not more advanced than that in Mild, and Marked indicates that the atrophy of the lobule is not more advanced than that in Moderate.

It is generally known that both the GTC region and the suspected lesion region A are depicted as the low-echo regions in the ultrasonic image U. For example, in "Su Hyun Lee et al. "Glandular Tissue Component and Breast Cancer Risk in Mammographically Dense Breasts at Screening Breast US", Radiology, Volume 301, October 1, 2021", it is disclosed that an examiner manually determines the GTC region and the suspected lesion region A and manually calculates the ratio of the GTC region, but it is difficult to distinguish the GTC region and the suspected lesion region A with high accuracy, and thus there may be a case in which the risk of cancer in the mammary gland region M cannot be considered with high accuracy. The evaluation unit 29 in the ultrasonic diagnostic apparatus according to the embodiment of the present invention performs the GTC evaluation based on the evaluation target region C obtained by excluding the suspected lesion region A from the mammary gland region M, and thus a highly accurate evaluation result ER can be output.

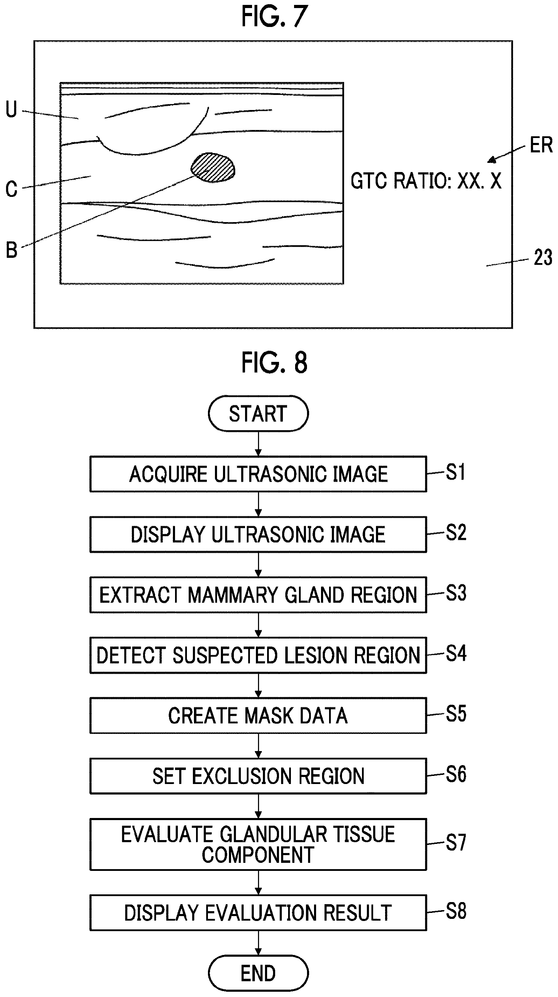

The display control unit 22 performs predetermined processing on the ultrasonic image U transmitted from the image generation unit 21 under the control of the body control unit 31, and displays the ultrasonic image U on the monitor 23. Further, for example, as shown in Fig. 7, the display control unit 22 highlights the exclusion region B set by the exclusion region setting unit 28 with respect to the evaluation target region C, and displays the evaluation result ER by the evaluation unit 29 on the monitor 23. In the example of Fig. 7, the ultrasonic image U on which the exclusion region B is superimposed and the ratio of the GTC region in the evaluation result ER are displayed on the monitor 23. The user can confirm the display to ascertain that the GTC evaluation is performed after the exclusion region B is set for the ultrasonic image U and the accurate evaluation result ER is output, so that it is possible to consider the risk of cancer in the breast of the subject with high accuracy even in a case in which the suspected lesion region A is present.

The display control unit 22 can highlight the exclusion region B by any method such as giving a color different from the surrounding color to the exclusion region B, displaying a contour line of the exclusion region B in a thick manner, or blinking the exclusion region B.

The monitor 23 displays the ultrasonic image U and the like under the control of the display control unit 22, and includes, for example, a display device such as a liquid crystal display (LCD) or an organic electroluminescence display (organic EL display).

The evaluation result memory 30 stores the evaluation result ER of the GTC evaluation for each partial region performed by the evaluation unit 29. For example, the user can read out the evaluation result ER through the input device 32 after the examination of the subject and consider the risk of cancer in the breast of the subject based on the evaluation result ER. As the evaluation result memory 30, for example, a recording medium such as a flash memory, an HDD, an SSD, an FD, an MO disc, an MT, a RAM, a CD, a DVD, an SD card, or an USB memory can be used.

The body control unit 31 controls each unit of the apparatus body 2 and the transmission-and-reception circuit 12 of the ultrasonic probe 1 based on a control program or the like, which is stored in advance.

The input device 32 is a device used by the user to perform an input operation, and includes, for example, devices such as a keyboard, a mouse, a trackball, a touch pad, and a touch sensor provided in a superimposed manner on the monitor 23.

The processor 34 including the image generation unit 21, the display control unit 22, the mammary gland region extraction unit 25, the lesion detection unit 26, the mask data creation unit 27, the exclusion region setting unit 28, the evaluation unit 29, and the body control unit 31 is configured by a central processing unit (CPU) and a control program for causing the CPU to execute various kinds of processing, but the processor 34 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the image generation unit 21, the display control unit 22, the mammary gland region extraction unit 25, the lesion detection unit 26, the mask data creation unit 27, the exclusion region setting unit 28, the evaluation unit 29, and the body control unit 31 of the processor 34 can also be configured by being integrated partially or entirely into one CPU or the like.

Next, an operation of the ultrasonic diagnostic apparatus according to the embodiment will be described with reference to a flowchart shown in Fig. 8.

First, in step S1, the breast of the subject is imaged by using the ultrasonic probe 1, and the ultrasonic image U is acquired. In this case, under the control of the body control unit 31, the transmission and reception of the ultrasonic waves from the plurality of transducers of the transducer array 11 are started in accordance with the drive signal from the pulser 13 of the transmission-and-reception circuit 12 of the ultrasonic probe 1, the ultrasonic echo from the inside of the breast of the subject is received by the plurality of transducers of the transducer array 11, the reception signal which is an analog signal is output to the amplifying unit 14 and is amplified by the amplifying unit 14, and the amplified reception signal is AD-converted by the AD conversion unit 15 to acquire the reception data.

The reception focus processing is performed on the reception data by the beam former 16, and the sound ray signal generated by the reception focus processing is transmitted to the image generation unit 21 of the apparatus body 2, and as a result, the ultrasonic image U representing the tomographic image information of the breast of the subject is generated by the image generation unit 21. In this case, the signal processing unit 41 of the image generation unit 21 performs the correction of the attenuation in accordance with the depth of the reflection position of the ultrasonic wave and the envelope detection processing on the sound ray signal, the DSC 42 performs the conversion into the image signal in accordance with the normal television signal scanning method, and the image processing unit 43 performs various types of necessary image processing such as gradation processing.

Next, in step S2, the ultrasonic image U generated by the image generation unit 21 is displayed on the monitor 23 via the display control unit 22, and is stored in the image memory 24.

In a case of acquiring the ultrasonic image U, the transmission intensity of the ultrasonic wave and the depth range of the ultrasonic image U displayed on the monitor 23 are adjusted under the control of the body control unit 31 such that the entire breast of the subject, that is, for example, the breast region BR of the subject shown in Fig. 4 or Fig. 6 is within the screen.

In subsequent step S3, the mammary gland region extraction unit 25 detects the breast region BR of the subject from the ultrasonic image U acquired in step S1, and extracts the mammary gland region M from the detected breast region BR. The mammary gland region extraction unit 25 can perform the image recognition using at least one of template matching, an image analysis technique using a feature value, such as AdaBoost, SVM, or SIFT, or a determination model that has been trained using a machine learning technique such as deep learning, in order to detect the breast region BR and to extract the mammary gland region M, for example.

In step S4, the lesion detection unit 26 detects the suspected lesion region A in the mammary gland region M extracted in step S3 based on the ultrasonic image U acquired in step S1. The lesion detection unit 26 can detect the suspected lesion region A by using, for example, at least one of template matching, an image analysis technique using a feature value, such as AdaBoost, SVM, or SIFT, or a determination model that has been trained by using a machine learning technique such as deep learning.

In step S5, the mask data creation unit 27 creates the mask data MD1 of the suspected lesion region A detected in step S4 as shown in Fig. 5. The mask data creation unit 27 can create, for example, data having the same size and shape as those of the suspected lesion region A detected in step S4 and having the same positional information as the positional information of the suspected lesion region A in the ultrasonic image, as the mask data MD1.

In step S6, as shown in Fig. 6, the exclusion region setting unit 28 sets the exclusion region B to be excluded from the target of the GTC evaluation performed by the evaluation unit 29, based on the mask data MD1 created by the mask data creation unit 27.

In step S7, the evaluation unit 29 extracts the GTC region from the evaluation target region C obtained by excluding the exclusion region B set in step S6 from the mammary gland region M extracted in step S3, calculates the ratio of the GTC region in the evaluation target region C, and performs the GTC evaluation based on the calculated ratio of the GTC region.

The evaluation unit 29 can distinguish the GTC region and the edematous stroma in the evaluation target region C from each other and extract the GTC region by, for example, binarizing the mammary gland region M of the ultrasonic image U using the brightness threshold value Thb. In addition, the evaluation unit 29 can extract the GTC region using the trained model that has learned the GTC region (segmentation) in the mammary gland region M in the training ultrasonic image in which the breast is imaged, in machine learning such as deep learning.

In addition, the evaluation unit 29 can calculate the GTC region ratio, for example, by a ratio of the sum of the number of pixels occupied by all the low-echo regions in the evaluation target region C to the number of pixels occupied by the high-echo region in the evaluation target region C in the ultrasonic image U. The evaluation unit 29 can use, for example, the ratio of the GTC region in the evaluation target region C calculated as described above as the evaluation result ER.

In addition, for example, the evaluation unit 29 can also determine the category of the GTC region based on the ratio of the GTC region in the evaluation target region C and use the category as the evaluation result ER. In this case, the evaluation unit 29 can output any one of a plurality of predetermined categories, for example, the two categories of Low and High, or the four categories of Minimal, Mild, Moderate, and Marked, as the category of the GTC region.

It is generally known that both the GTC region and the suspected lesion region A are depicted as the low-echo regions in the ultrasonic image U, but the evaluation unit 29 performs the GTC evaluation based on the evaluation target region C obtained by excluding the suspected lesion region A from the mammary gland region M, and thus the evaluation unit 29 can output a highly accurate evaluation result ER.

At last, in step S8, the display control unit 22 displays the evaluation result ER of the GTC evaluation output in step S7 on the monitor 23, for example, as shown in Fig. 7. In this case, the display control unit 22 can highlight the exclusion region B set by the exclusion region setting unit 28 with respect to the evaluation target region C and display the evaluation result ER obtained by the evaluation unit 29 on the monitor 23.

The user can confirm the display to ascertain that the GTC evaluation is performed after the exclusion region B is set for the ultrasonic image U and the accurate evaluation result ER is output, so that it is possible to consider the risk of cancer in the breast of the subject with high accuracy even in a case in which the suspected lesion region A is present.

In a case in which the processing of step S8 is completed in this manner, the operation of the ultrasonic diagnostic apparatus according to the flowchart of Fig. 8 is completed.

As described above, with the ultrasonic diagnostic apparatus according to the embodiment, the lesion detection unit 26 detects the suspected lesion region A in the mammary gland region M based on the ultrasonic image U in which the mammary gland region M of the subject is imaged, the mask data creation unit 27 creates the mask data MD1 of the suspected lesion region A detected by the lesion detection unit 26, the exclusion region setting unit 28 sets the exclusion region B to be excluded from the target of the GTC evaluation based on the mask data MD1, and the evaluation unit 29 performs the GTC evaluation on the evaluation target region C excluding the exclusion region B set by the exclusion region setting unit 28 from the mammary gland region M, so that an accurate evaluation result ER of the GTC evaluation can be output and the user can accurately consider the risk of cancer in the breast of the subject even in a case in which the suspected lesion region A is present.

In addition, a case has been described in which the transmission-and-reception circuit 12 is provided in the ultrasonic probe 1, but the transmission-and-reception circuit 12 may be provided in the apparatus body 2.

Furthermore, a case has been described in which the image generation unit 21 is provided in the apparatus body 2, but the image generation unit 21 may be provided in the ultrasonic probe 1.

Moreover, the apparatus body 2 may be a so-called stationary type, a portable type that is easy to carry, or a so-called handheld type that is configured by, for example, a smartphone or a tablet type computer. As described above, the type of the device constituting the apparatus body 2 is not particularly limited.

In addition, a case has been described in which the ultrasonic probe 1 and the apparatus body 2 are connected to each other in a wired manner, but the ultrasonic probe 1 and the apparatus body 2 may be connected to each other in a wireless manner.

In addition, although it has been described that the lesion detection unit 26 detects the suspected lesion region A from the ultrasonic image U, for example, in a case in which the edge of the suspected lesion region A is unclear for some reason, it is considered that the reliability degree of detection is inferior to a case in which the edge of the suspected lesion region A is clearly depicted. Therefore, the lesion detection unit 26 can calculate the reliability degree of the detection of the suspected lesion region A based on, for example, the sharpness of the edge of the suspected lesion region A, the overall contrast of the ultrasonic image U, and the like. In addition, in a case in which the lesion detection unit 26 performs the processing of detecting the suspected lesion region A by a machine learning method, the lesion detection unit 26 can also calculate a similarity between a pattern of the trained suspected lesion region A and a pattern of the suspected lesion region A detected from the ultrasonic image U as the reliability degree of the detection.

In this case, the display control unit 22 can display the exclusion region B on the monitor 23 in a color or a form in accordance with the reliability of the detection of the suspected lesion region A in the lesion detection unit 26, such as displaying the exclusion region B in red in a case in which the reliability degree is 80% or more, displaying the exclusion region B in yellow in a case in which the reliability degree is in a range of 50% or more and less than 80%, and displaying the exclusion region B in blue in a case in which the reliability degree is less than 50%. The user can consider the risk of cancer in the breast of the subject in detail by confirming the evaluation result of the GTC evaluation displayed on the monitor 23 with reference to the display form of the exclusion region B.

In addition, in a case in which the reliability degree of the detection of the suspected lesion region A is decreased for some reason, the user can also correct or delete the exclusion region B by, for example, the input operation using the input device 32. In this case, the display control unit 22 can display a dialog for confirming with the user whether to correct or delete the exclusion region B on the monitor 23. The user can confirm the dialog and then issue an instruction to correct or delete the exclusion region B or an instruction to maintain the current exclusion region B.

In addition, in a case in which the dimension of the mask data MD1 created by the mask data creation unit 27 in the ultrasonic image U is extremely small, even in a case in which the exclusion region B is set based on the mask data MD1, the evaluation result ER equivalent to the evaluation result ER of the GTC evaluation in a case in which the exclusion region B is not set can be obtained, so that the exclusion region setting unit 28 can not set the exclusion region B in a case in which the mask data MD1 is smaller than a predetermined first size threshold value. As a result, it is possible to save a calculation load in the ultrasonic diagnostic apparatus in a case of setting the exclusion region B and to reduce power consumption in the ultrasonic diagnostic apparatus.

In a case in which the dimension of the mask data MD1 created by the mask data creation unit 27 in the ultrasonic image U is extremely large, the suspected lesion region A is extremely large, and there is a probability that a disease state is progressing as a more detailed examination or treatment is required in the breast of the subject. Therefore, in a case in which the mask data MD1 is larger than a predetermined second size threshold value, the evaluation unit 29 can notify the user of that the suspected lesion region A is very large by a message or the like displayed on the monitor 23 without performing the GTC evaluation.

In addition to the size of the mask data MD1, the evaluation unit 29 can also calculate a degree of progression of the disease state of the subject using any method, such as a method based on a brightness distribution of the ultrasonic image U at the position corresponding to the mask data MD1 or a ratio between the mammary gland region M and the suspected lesion region A. In a case in which the calculated degree of progression of the disease state exceeds a predetermined threshold value, the evaluation unit 29 can notify the user of the degree of progression of the disease state of the subject without performing the GTC evaluation.

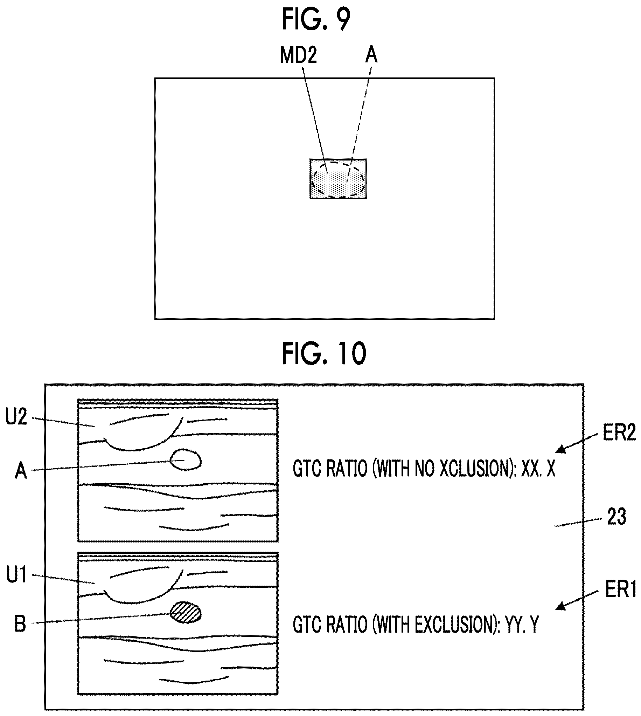

In addition, although it has been described that the mask data creation unit 27 creates the mask data MD1 having the same size and shape as those of the suspected lesion region A, the size and shape of the mask data MD1 created by the mask data creation unit 27 are not particularly limited as long as the suspected lesion region A can be surrounded. For example, as shown in Fig. 9, the mask data creation unit 27 can also create rectangular mask data MD2 that surrounds the suspected lesion region A. In this case, the mask data MD2 can be composed of matrix data including coordinates of four vertices of a rectangle in the ultrasonic image U.

In addition, the evaluation unit 29 can also perform the GTC evaluation on the mammary gland region M in which the exclusion region B is not excluded, in addition to the GTC evaluation on the evaluation target region C. In this case, for example, as shown in Fig. 10, the display control unit 22 can display the evaluation result ER1 of the GTC evaluation on the evaluation target region C and the evaluation result ER2 of the GTC evaluation on the mammary gland region M in which the exclusion region B is not excluded, on the monitor 23. In the example of Fig. 10, an ultrasonic image U1 in which the exclusion region B is removed and an ultrasonic image U2 in which the suspected lesion region A is shown without removing the exclusion region B are further displayed on the monitor 23. For example, in a case in which it is difficult to distinguish between the suspected lesion region A and the GTC region, the user can more specifically consider the risk of cancer in the breast of the subject by confirming the evaluation results ER1 and ER2.

In addition, although it has been described that the two-dimensional ultrasonic image U is generated by the image generation unit 21, the image generation unit 21 can also generate a so-called three-dimensional ultrasonic image based on the ultrasonic images U of a plurality of consecutive frames. The mammary gland region extraction unit 25 can extract a three-dimensional mammary gland region M from the three-dimensional ultrasonic image. The lesion detection unit 26 can detect a three-dimensional suspected lesion region A included in the three-dimensional mammary gland region M. The mask data creation unit 27 can create three-dimensional mask data MD1 which covers the three-dimensional suspected lesion region A. The exclusion region setting unit 28 can set a three-dimensional exclusion region B based on the three-dimensional mask data MD1. The evaluation unit 29 can perform the GTC evaluation based on the three-dimensional ultrasonic image, that is, in the three-dimensional evaluation target region C obtained by excluding the three-dimensional exclusion region B from the three-dimensional mammary gland region M.

In this way, by performing the GTC evaluation via the evaluation unit 29 based on the three-dimensional ultrasonic image, a more detailed and accurate evaluation result ER of the GTC evaluation is obtained, so that the user can consider the risk of cancer in the breast of the subject with higher accuracy.

Further, although it has been described that the evaluation result ER of the GTC evaluation output by the evaluation unit 29 is stored in the evaluation result memory 30, the evaluation result ER can also be stored in association with the ultrasonic image U used for the GTC evaluation.

In addition, the apparatus body 2 can also comprise a transmission circuit (not shown) that transmits the evaluation result ER of the GTC evaluation output by the evaluation unit 29 to an external server device (not shown) such as an examination information management system such as a so-called electronic medical record, a report system that creates a report using a medical image, and a picture archiving and communication system (PACS). In a case of transmitting the evaluation result ER to the external server device or the like, for example, a protocol, such as hypertext transfer protocol (HTTP), hypertext transfer protocol secure (HTTPS), file transfer protocol (FTP), health level seven (HL7), or digital imaging and communications in medicine (DICOM), can be used.

### Explanation of References

1: ultrasonic probe
2: apparatus body
11: transducer array
12: transmission-and-reception circuit
13: pulser
14: amplifying unit
15: AD conversion unit
16: beam former
21: image generation unit
22: display control unit
23: monitor
24: image memory
25: mammary gland region extraction unit
26: lesion detection unit
27: mask data creation unit
28: exclusion region setting unit
29: evaluation unit
30: evaluation result memory
31: body control unit
32: input device
33: image acquisition unit
34: processor
41: signal processing unit
42: DSC
43: image processing unit
A: suspected lesion region
B: exclusion region
BR: breast region
C: evaluation target region
ER, ER1, ER2: evaluation result
L1: front boundary line
L2: rear boundary line
M: mammary gland region
MD1, MD2: mask data
S: skin
T: pectoralis major
U, U1, U2: ultrasonic image

## Claims

1. An ultrasonic diagnostic apparatus comprising:
a lesion detection unit that detects a suspected lesion region in a mammary gland region of a subject based on an ultrasonic image in which the mammary gland region is imaged;
a mask data creation unit that creates mask data of the suspected lesion region detected by the lesion detection unit;
an exclusion region setting unit that sets an exclusion region to be excluded from a target of a glandular tissue component evaluation based on the mask data; and
an evaluation unit that performs the glandular tissue component evaluation on an evaluation target region obtained by excluding the exclusion region set by the exclusion region setting unit from the mammary gland region.

2. The ultrasonic diagnostic apparatus according to claim 1,
wherein the evaluation unit classifies the evaluation target region into a low-echo region and a high-echo region based on a predetermined brightness threshold value, and outputs a ratio between the number of pixels occupied by the low-echo region and the number of pixels occupied by the high-echo region as a result of the glandular tissue component evaluation.

3. The ultrasonic diagnostic apparatus according to claim 1,
wherein the evaluation unit determines a category of a glandular tissue component in the mammary gland region based on the ultrasonic image including the evaluation target region, and outputs the category as a result of the glandular tissue component evaluation.

4. The ultrasonic diagnostic apparatus according to claim 3,
wherein the evaluation unit determines the category of the glandular tissue component using a trained model that has been trained through machine learning based on a plurality of training data each of which includes the ultrasonic image in which the mammary gland region is imaged and the category of the glandular tissue component in the mammary gland region.

5. The ultrasonic diagnostic apparatus according to claim 1, further comprising:
a monitor; and
a display control unit that displays the ultrasonic image on the monitor,
wherein the display control unit highlights the exclusion region set by the exclusion region setting unit on the monitor.

6. The ultrasonic diagnostic apparatus according to claim 5,
wherein the display control unit highlights the exclusion region with respect to the evaluation target region on the monitor.

7. The ultrasonic diagnostic apparatus according to claim 5,
wherein the display control unit displays the exclusion region on the monitor in a color or a form in accordance with a reliability degree of the detection of the suspected lesion region performed by the lesion detection unit.

8. The ultrasonic diagnostic apparatus according to claim 5,
wherein the display control unit displays a dialog for confirming with a user whether to correct or delete the exclusion region on the monitor.

9. The ultrasonic diagnostic apparatus according to claim 1,
wherein the exclusion region setting unit does not set the exclusion region in a case in which the mask data is smaller than a predetermined first size threshold value.

10. The ultrasonic diagnostic apparatus according to claim 1,
wherein the evaluation unit does not perform the glandular tissue component evaluation in a case in which the mask data is larger than a predetermined second size threshold value.

11. The ultrasonic diagnostic apparatus according to claim 5,
wherein the evaluation unit performs the glandular tissue component evaluation on the mammary gland region that does not exclude the exclusion region, in addition to the glandular tissue component evaluation on the evaluation target region, and
the display control unit displays a result of the glandular tissue component evaluation on the evaluation target region and a result of the glandular tissue component evaluation on the mammary gland region that does not exclude the exclusion region on the monitor.

12. The ultrasonic diagnostic apparatus according to claim 1,
wherein the lesion detection unit detects the suspected lesion region using a trained model that has been trained through machine learning based on a plurality of training data each of which includes the ultrasonic image in which the mammary gland region including the suspected lesion region is imaged.

13. The ultrasonic diagnostic apparatus according to claim 1,
wherein the lesion detection unit detects the suspected lesion region by image-analyzing the ultrasonic image.

14. The ultrasonic diagnostic apparatus according to claim 1,
wherein the ultrasonic image is a three-dimensional ultrasonic image, and
the evaluation unit performs the glandular tissue component evaluation based on the three-dimensional ultrasonic image.

15. A method of controlling an ultrasonic diagnostic apparatus, the method comprising:
detecting a suspected lesion region in a mammary gland region of a subject based on an ultrasonic image in which the mammary gland region is imaged;
creating mask data of the detected suspected lesion region;
setting an exclusion region to be excluded from a target of a glandular tissue component evaluation based on the mask data; and
performing the glandular tissue component evaluation on an evaluation target region obtained by excluding the exclusion region from the mammary gland region.
